# EUROPEAN PATENT SPECIFICATION

(11) **EP 3 095 422 B1**
(45) Date of publication and mention of the grant of the patent: **24.04.2019**
(21) Application number: 15305774.0
(22) Date of filing: 22.05.2015
(51) Int. Cl.: A61F 13/00, A61K 8/02, D04H 1/02, D04H 1/425

(54) **COTTON PAD, PROCESS AND DEVICE FOR ITS MANUFACTURE**
WATTEPAD, VERFAHREN ZU DESSEN HERSTELLUNG
TAMPON DE COTON, PROCÉDÉ ET DISPOSITIF POUR SA FABRICATION

(43) Date of publication of application: 23.11.2016
(73) Proprietor: Essity Operations France, 93400 Saint-Ouen (FR)
(72) Inventor: Gregoire, Philippe, 27700 LES ANDELYS (FR)
(74) Representative: Gevers & Orès

(56) References cited:
- WO-A1-94/17235
- CA-A1- 2 041 278
- GB-A- 396 418
- JP-A- 2014 210 073
- KR-A- 20000 061 027

## Description

The invention relates to cotton pads and to processes for manufacturing these cotton pads. It also relates to devices for implementing these processes.

In the present text, the terms "cotton pad(s)" are used to encompass any products into a given format and substantially containing cotton fibers in a proportion of from 55 to 100 weight (wt) % and artificial and/or synthetic and/or natural (other than cotton) fibers in a proportion of 0 to 45 wt %.

Examples of synthetic fibers which may be used are polyolefin-based heat melting fibers or polyester fibers.

Artificial fibers are manufactured from natural products which have been chemically modified.

Examples of artificial fibers are viscose fibers, and fibers called "bamboo fibers", etc...

Natural fibers are obtained from natural products which have not been chemically modified.

Examples of natural fibers are linen fibers.

The cotton pads are generally produced by cutting individual pads in a web of fibers. This web of fibers can be manufactured as described in European patent n° 0 681 621 or European patent application n°0 735 175 or PCT application WO 01/42548.

Skin care includes body care, face care in particular, care involving cosmetics namely face make-up and make-up removal, baby care namely cleaning and changing the infant, and the like.

Most hydrophilic cotton products or pads on the market are cut into formats, for example, circular (make-up remover disk), oval, or square or rectangular.

JP20144210073 discloses mascara removing cotton piece comprising cutting perforations for enabling a user to divide the cotton piece into a large cotton piece and a small cotton piece.

GB396418 discloses a cotton roll having perforations for enabling a user to subdivide it.

KR1020000061027 discloses a cotton roll having heat sealing part in order to be cut to the desired size by a user.

WO94/17235 discloses in figure 2 , an intermediate step of manufacture of a cotton pad. In this step, a cotton web comprising cut parts is manufactured. Cotton pads are detached from this web. But, in this document, the cotton pads are not detached by the user but is a following step of manufacture of the cotton pad.

All these documents are mute about the dimensions of the cut parts.

The cotton pads frequently are a mixture of cotton fibers of different grades and/or are a mixture of cotton fibers and other fibers depending on the desired product or the particular manufacturing method.

Their outer side faces may be identical or different in structure and composition.

The circular pads, which are generally used for removing make-up, in particular of eyes, generally have a diameter of about 56-57 mm.

The oval pads are generally used for removing make-up and have generally a largest dimension of about 90 mm and a smallest dimension of about 70 mm.

The rectangular pads are generally used for baby care. They generally have a length of about 110 mm and a width of about 90 mm.

Other shapes of the pads are also possible, such as a drop shape, or square shape.

These dimensions are generally appropriate for their intended use, *i.e.* for removing eye make-up and/or face make-up, for baby cleaning and changing...

But, when the user desires, for example, removing nail varnish, or making make-up retouch, or cleaning folds of the skin of babies, these dimensions are too large and the user tears the cotton pad.

When doing that, the cotton pad of the prior art then presents free fibers that can enter the eyes or stay in the baby's folds, which is a source of irritations and pains.

Furthermore, the dimension of the obtained torn pieces may be inappropriate and, in any case, the part of the pad which is not used is difficult to keep for a further use.

The invention aims to palliate these drawbacks of the prior art cotton pads.

For attaining this aim, the invention proposes a cotton pad as defined in the appended claims, having at least one precutting line, this precutting line consisting of a succession of links and cut parts, delimiting at least two detachable parts of the pad, the two parts being maintained attached together by the links. The invention also proposes methods and devices for manufacturing such a cotton pad These methods and devices are as defined in the appended claims.

The invention will be better understood and other features and advantages of the invention will more clearly appear when reading the following description which is made in reference to the annexed figures in which:

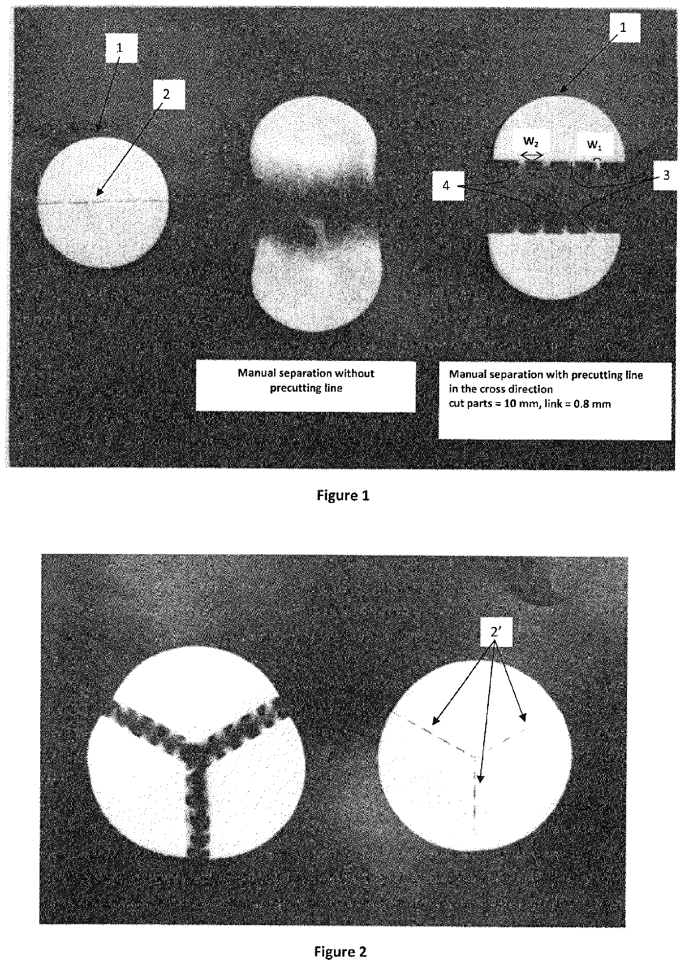

- **figure 1** shows:
   - on the left hand, a photograph of a circular pad according to the invention, having one precutting line formed in cross direction machine, in which the width of the cut portions is 10 mm and the width of the links is 0.8 mm. On this photograph, the precutting line has been rendered more visible by moving away the two parts of the cotton pad,
   - on the middle: a photograph of a cotton pad of the prior art torn into two parts, and
   - on the right hand, the cotton pad shown on the left end, but torn,
- **figure 2** shows a photograph of a cotton pad according to the invention comprising three cutting lines,
- **figure 3** shows a photograph of a cotton pad according to the invention having two precutting lines, perpendicular one to the other. On this photograph, the precutting line has been rendered more visible by moving away the two parts of the cotton pad,
- **figure 4** shows two round pads comprising one precutting line formed in the cross machine direction and having different widths of links, before having been torn and after having been torn,
- **figure 5** shows a photograph of a rotary cutting device for manufacturing the cotton pads of the invention,
- **figure 6** shows a slitting knives of a device of the prior art for slitting a cotton web A into cotton webs B having a smaller width than the cotton web B and a rotary cylinder providing a counter pressure to the slitting knife,
- **figure 7** schematically shows a device comprising a upper cylinder and a lower rotary cylinder situated face to face. The rotary cylinder provides a counter pressure to the knives of the upper cylinder. In this device, the knife holder carries two knives, one of the knives being not serrated and being used for slitting web A into webs B having a smaller width than web A and the other knife being a serrated knife for forming a precutting line into cotton webs B,
- **figure 8** shows, on the left hand, the non serrated cutting knife represented in figures 6 and 7, and, on the right hand, the serrated cutting knife shown in figure 7 which is to be placed between two non serrated knives represented in figures 6 and 7, for forming a precutting line in the machine direction of the cotton webs B,
- **figure 9** schematically shows a device for forming precutting lines in webs B. This device comprises firstly, an upper rotary cylinder comprising, knives for forming precutting lines in webs B, these knives being serrated, , these serrated knives forming an angle of 90° ± 1° with the axis of the rotary lower cylinder, and, secondly, a lower cylinder for providing a counter pressure to the knives,
- **figure 10** is a graph showing the variation of the average tensile strengths of a circular cotton pad of the invention made as described in EP 1 106 723 A1, having one precutting line formed in the cross machine direction, as a function of the width of the cut parts in the precutting line,
- **figure 11** is a graph showing the variation of the average tensile strengths as a function of the width of the cut parts of the precutting line formed in the machine direction in a circular cotton pad manufactured as described in EP 1 106 723 A1,
- **figure 12** is a graph showing the variation of the average tensile strengths of a circular cotton pad of the invention having one precutting line formed in the cross machine direction as a function of the width of the cut parts in the precutting line, the circular cotton pad being made as described in WO 94/17235,
- **figure 13** is a graph showing the variation of the average tensile strengths as a function of the width of the cut parts of the precutting line formed in the machine direction in a circular pad manufactured by the method described in WO 94/17235,

The cotton pad of the invention has, as the cotton pads of the prior art, two faces which can be used independently. But in contrast to the cotton pads of the prior art, it can be used by only portions of the two faces, without generating free fibers and deformation of the torn cotton parts and/or without wasting the rest of the cotton pad. Otherwise stated, the surface of use of each face of the cotton pad of the invention can be used in its entire without detachment along the precutting line(s) or can be used only by portions of surface, without production of free fibers.

The cotton pad of the invention will now be described with reference to figures 1, 2 and 3.

Figures 1, 2 and 3 show photographs of examples of cotton pads of the invention.

As one can see on figure 1, the cotton pad of the invention, noted 1 in figure 1, has at least one precutting line noted 2 in figure 1. More precisely, the cotton pad shown in figure 1 has one precutting line 2. This cutting line may be a straight line, as represented in figure 1, but it can have any other shape, such as a curved shape.

This precutting line 2 delimits two parts in the cotton pad.

The precutting line 2 comprises links, noted 3 in figure 1, having a width, noted w₁ in figure 1, and cut parts, noted 4 in figure 1, having a width noted w₂ in figure 1.

This precutting line 2 enables the user to tear the cotton pad into two parts while minimizing the zones of free fibers produced in the links of the cotton pad which is used.

Indeed, without precutting line, when the user wants to tear the cotton pad of the prior art into two parts, the user obtains two parts having free fibers, as shown in the center of the photograph represented in figure 1.

The cotton pad of the invention can have three precutting lines as shown in figure 2 in which they are noted 2', enabling the user to tear three parts in the cotton pad.

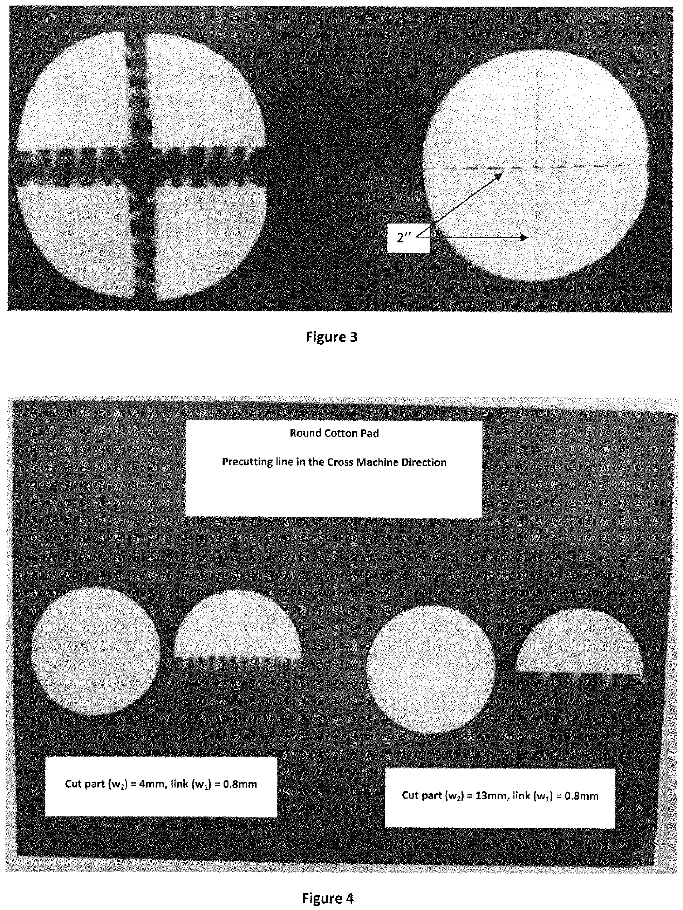

The cotton pad of the invention may also have two precutting lines, one crossing the other, as shown in figure 3, in which the precutting lines are noted 2" and enabling the user to separate the cotton pad in four portions.

Again, one can see on figures 2 and 3 that there are little free fibers produced in the zones corresponding to the links, in the obtained portions after tearing the cotton pad of the invention.

As one can see on figure 4, the amount of free fibers depends on the width w₁ of the links 3 and on the number of these links 3.

Thus, the width w₁ of the links 3 may vary from 0.3 to 1.3 mm. Preferably, the width w₁ of the links 3 is of 0.8 mm in order to obtain the best compromise between a minimum formation of free fibers and a minimum deformation of the torn parts as well as the maintenance of the integrity of the cotton pad when used not torn.

The width w₂ of the cut parts 4 may vary between 4 and 15 mm. Preferably, the width w₂ is of 10 mm again for obtaining the best compromise between the amount of free fibers formed and the maintenance of the integrity of the cotton pad when not torn.

The precutting lines 2, 2', 2" may be created in the direction machine of the cotton pad, *i.e.* in the direction in which the cotton web is driven during its manufacture.

But, preferably, the at least one precutting line is formed in the cross machine direction, *i.e.* perpendicular to the direction in which the cotton web is driven during its manufacture, *i.e.* the at least precutting line is formed with an angle of 90° ± 1° with the machine direction.

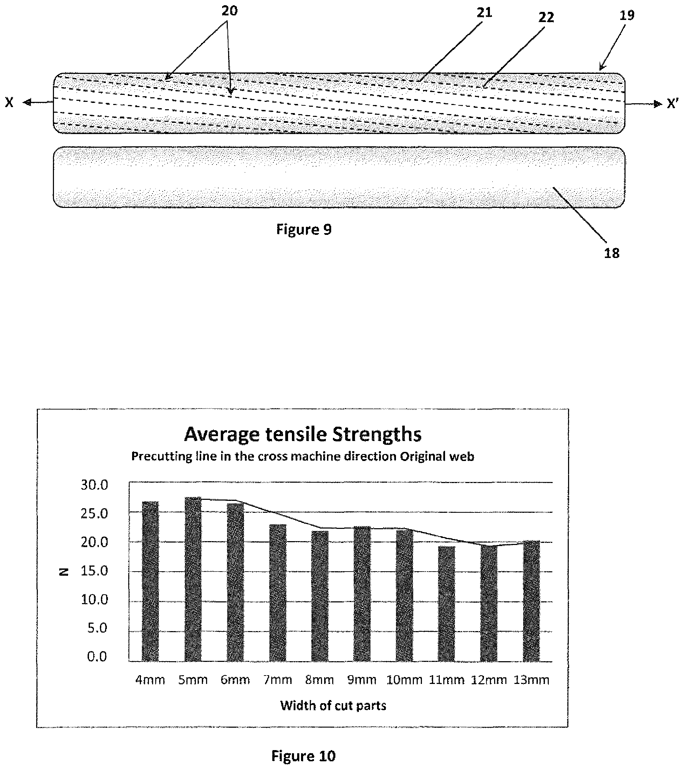

Indeed, as one can see from following table 1 and as shown in figure 10, which represents the average tensile strengths of cotton pads having one precutting line formed in the cross machine direction, this precutting line having widths w₂ of the cut parts varying from 4 to 13 mm, and widths w₁ of the links 3 being maintained constant at 0.8 mm, presents plateaus. Thus, when the width of the cut parts 4 is between about 4 and 6 mm, the average tensile strengths is of about 27 Newton, when the width w₂ of the cut part is comprised between 7 and 10 mm, the average tensile strengths is of about 22 Newton and when the width w₂ of the cut part is comprised between 11 and 13 mm, the average tensile strengths is of about 19.500 Newton.

This is very surprising and unexpected.

**Table 1:**

| Precutting in the cross direction machine | | | |
|---|---|---|---|
| Round cotton pads (Demak UP® ORIGINAL) | | | |
| **w₂** (mm) | **Tensile strength measured in the machine direction N** | **Tensile strength measured in the cross machine direction N** | **Average tensile strength N** |
| 0 | 53.40 | 32.60 | 43.00 |
| 4 | 19.50 | 34.00 | 26.75 |
| 5 | 17.50 | 37.50 | 27.50 |
| 6 | 14.70 | 38.20 | 26.45 |
| 7 | 13.30 | 32.50 | 22.90 |
| 8 | 11.30 | 32.40 | 21.85 |
| 9 | 10.20 | 35.00 | 22.60 |
| 10 | 7.9 | 36.00 | 21.95 |
| 11 | 7.7 | 30.90 | 19.30 |
| 12 | 7.4 | 31.40 | 19.40 |
| 13 | 7.1 | 33.40 | 20.25 |

The average tensile strengths reported in Table 1 and figure 10 are the average values of tensile strengths measured by the test disclosed in the following description and obtained on ten samples having one precutting line formed in the cross machine direction.

These samples are circular cotton pads of 56 mm of diameter made in a web of cotton fibers manufactured according to the method described in EP 1 106 723 A1 sold under the trademark "Demak UP® ORIGINAL".

The tensile strengths are measured both in the machine direction and the cross machine direction. Indeed, measuring both strengths is important because the user must have the possibility to take the cotton pad and use it, both in the machine direction and the cross direction machine.

The average tensile strength is the sums of the tensile strength measured in the machine direction and of the tensile strength measured in the cross machine direction divided by 2.

The test method is a test measuring the tensile strength of non woven samples. This test method is based on the EDANA TENSILE STRENGTH method number 20.2-89 dated February 99 which has been modified.

More precisely, this EDANA TENSILE STRENGTH test method has been modified as follows:
1) it was carried out on the entire cotton pad and not, as stated in the EDANA TENSILE STRENGTH test method on a sample having a width of 50 mm and a length of more than 200 mm, and
2) the testing jaws where set at 30 mm apart instead of 200 mm.

Indeed, the tested cotton pads of the invention having a diameter of 56-57 mm, it was not possible to cut samples having a width of 50 mm along an entire length of more than 200 mm.

The tensile strength, *i.e.* the maximum force measured before the sample brakes of each sample has been measured along the direction machine production of the sample and along the cross direction machine production of the sample.

In all the samples, *i.e.* the circular, the oval and the rectangular cotton pads, manufactured from a cotton web having a weight per unit area comprised between 150 and 400 g/m² (respectively sold under the trademark "Demak UP® ORIGINAL" manufactured as described in EP 1 106 723 A1, and sold under the trademark "Demak UP® SENSITIVE" manufactured as described in WO 94/17235 and sold under the trademark "Lotus® Baby" manufactured as described in EP 1 167 605 A1), the same results have been obtained: the average tensile strengths exhibited plateaus when at least one of the precutting line(s) was formed in the cross machine direction.

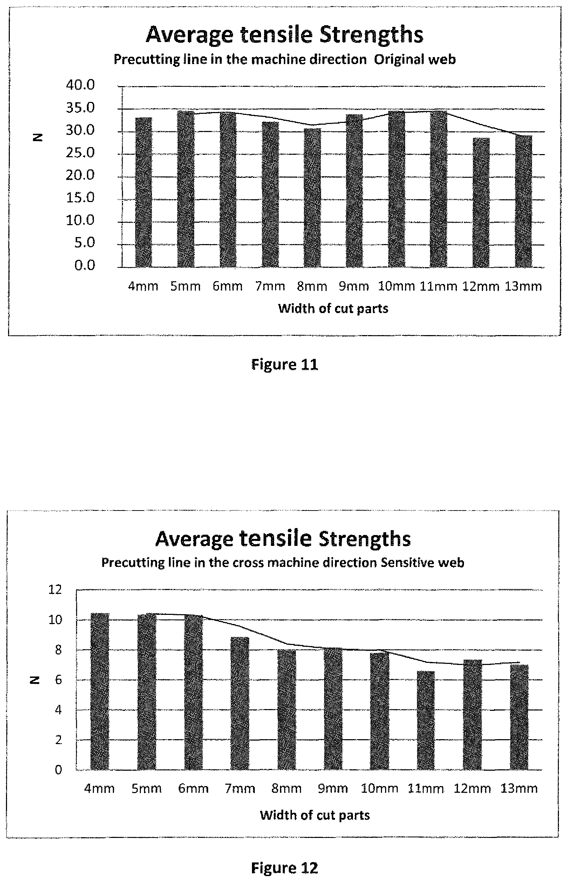

Thus, the average tensile strengths obtained on circular cotton pads Demak UP® SENSITIVE having one precutting line formed in the cross machine direction are reported in following table 2 and in figure 12.

**Table 2:**

| Precutting line in the cross direction machine | | | |
|---|---|---|---|
| Round cotton pads - Demak UP® SENSITIVE | | | |
| **w₂** (mm) | **Tensile strength measured in the machine direction N** | **Tensile strength measured in the cross machine direction N** | **Average tensile strength N** |
| 0 | 23.2 | 12.6 | 17.9 |
| 4 | 7.9 | 13.0 | 10.45 |
| 5 | 7.7 | 13.0 | 10.35 |
| 6 | 6.8 | 13.8 | 10.30 |
| 7 | 5.7 | 12.0 | 8.85 |
| 8 | 4.5 | 11.4 | 7.95 |
| 9 | 4.2 | 12.1 | 8.15 |
| 10 | 3.2 | 12.4 | 7.8 |
| 11 | 3.0 | 10.2 | 6.6 |
| 12 | 3.4 | 11.3 | 7.35 |
| 13 | 2.8 | 11.2 | 7.00 |

The tested samples were also circular cotton pads of 56 mm of diameter made in a web of cotton fibers manufactured according to the method described in EP 1 106 723 A1 sold under the trademark "Demak Up® ORIGINAL".

**Table 3:**

| Precutting line in the direction machine | | | |
|---|---|---|---|
| Round cotton pads-Demak Up® ORIGINAL | | | |
| **w₂** (mm) | **Tensile strength measured in the machine direction N** | **Tensile strength measured in the cross machine direction N** | **Average tensile strength N** |
| 0 | 53.40 | 32.60 | 43.00 |
| 4 | 54.10 | 12.20 | 33.15 |
| 5 | 58.10 | 11.20 | 34.65 |
| 6 | 58.00 | 10.20 | 34.10 |
| 7 | 56.80 | 7.60 | 32.20 |
| 8 | 55.40 | 6.10 | 30.75 |
| 9 | 61.60 | 6.10 | 38.85 |
| 10 | 61.90 | 7.30 | 34.60 |
| 11 | 63.90 | 5.30 | 34.60 |
| 12 | 53.20 | 4.20 | 28.70 |
| 13 | 54.10 | 4.40 | 29.25 |

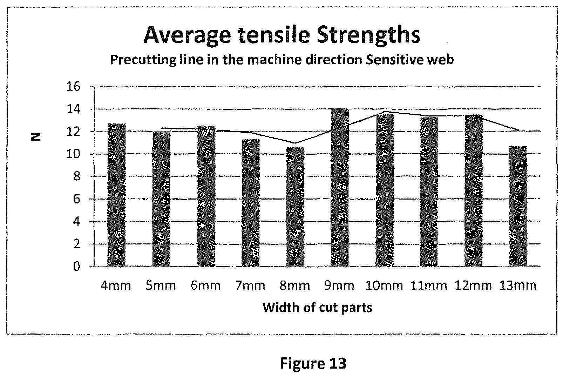

In contrast, as shown in following tables 3 and 4 and in figures 11 and 13, when the precutting line(s) is(are) formed only in the direction machine, the average tensile strengths, as a function of the width w₂ of the cut parts of the precutting line(s) do not present plateaus and are in general higher than the average tensile strengths obtained with the pad having a precutting line formed in the cross machine direction.

**Table 4:**

| Precutting line in the direction machine | | | |
|---|---|---|---|
| Round cotton pads- Demak Up® SENSITIVE | | | |
| **w₂** (mm) | **Tensile strength measured in the machine direction N** | **Tensile strength measured in the cross machine direction N** | **Average tensile strength N** |
| 0 | 23.2 | 12.6 | 17.9 |
| 4 | 20.1 | 5.3 | 12.7 |
| 5 | 19.9 | 3.9 | 11.9 |
| 6 | 21.1 | 3.9 | 12.5 |
| 7 | 19.2 | 3.4 | 11.3 |
| 8 | 18.6 | 2.6 | 10.6 |
| 9 | 25.2 | 2.9 | 14.05 |
| 10 | 23.6 | 3.4 | 13.5 |
| 11 | 23.9 | 2.6 | 13.25 |
| 12 | 24.6 | 2.4 | 13.50 |
| 13 | 19.2 | 2.2 | 10.70 |

The tested samples were also circular cotton pads of 56 mm of diameter made in a web of cotton fibers manufactured according to the method described in EP 1 106 723 A1 for table 3 and as described in WO 94/17235.

The average tensile strengths obtained when the precutting line(s) is(are) formed in the direction machine do not present any plateau and may increase or decrease in a random manner. In that case, the average tensile strengths depend from other factors than the precutting line(s) or the dimensions of the cut parts and links. They could depend, for example, on the regularity of the distribution of the fibers in the starting web. This distribution depends on the means for forming the web (aerolic, pneumatic, etc...).

Consequently, obtaining pads having a regular and uniform strength for a width of the cut parts varying between 7 and 10 mm is easier when the precutting line(s) is(are) formed in the cross machine direction.

However, because the average tensile strengths of the cotton pads according to the invention are generally higher, when the precutting line(s) is(are) formed in the machine direction, such cotton pads are also appropriate for the aims of providing cotton pads which can be separated, when desired by the user, in smaller formats without difficulty and without obtaining free fibers.

The fact that when the at least one precutting line is formed in the cross machine direction the average tensile strengths present plateaus with different widths of the cut parts in the precutting line enables, on the one hand, to minimize the amount of formed free fibers because the width of the cut parts may be large so that there is less links producing free fibers, and, on the other hand, the strength obtained with the wider cut parts is sufficient for permitting the user to use the entire cotton pad without risks of accidentally tearing it and also to maintain the integrity of the cotton pad in use, when used in its entirety, and to avoid to have make-up, or cleaning products, lost through the "holes" of the cut parts in the cotton pad.

Furthermore, the cotton pads of the invention can be impregnated before or after their manufacture. The composition with which the cotton pads of the invention are impregnated can be a cosmetic skin care composition, *i.e.* a cleaning or cosmetic composition. It can also be a pharmaceutical composition.

The invention also provides a method for producing a cotton pad according to the invention.

This method comprises the following steps:
a) providing a cotton web roll,
b) unwinding the cotton web roll of step a),
c) cutting the cotton web obtained in step b) into cotton pads having the desired shape and dimensions.

But, the method of the invention comprises a supplementary step d) of creating at least one precutting line throughout the cotton pad, preferably in the cross machine direction.

The method of the invention can also comprise before step a) the steps of: a1) manufacturing a cotton web A (cotton lap) having a width L1 from the desired fibers, and then a2) slitting this cotton web A into cotton webs B having a width L2 smaller than the width L1 of the cotton web A and winding webs B into cotton web rolls.

The desired fibers may be 100 wt % cotton fibers or may be a mixture comprising from 55 to 100 wt % cotton fibers and from 45 to 0 wt % artificial and/or synthetic and/or natural fibers.

In the method of the invention, steps c) and d) of cutting the cotton webs B into cotton pads and creating at least one precutting line throughout these cotton pads may be carried out simultaneously.

But the at least one precutting line may be created in the cotton webs B before step c) of cutting these cotton webs into the cotton pads and after step b).

Step d) of creating the at least one precutting line in the cotton pads may also be carried out simultaneously with step a2). This embodiment of the process of the invention is particularly advantageous when the at least on precutting line is formed in the machine direction because it necessitates only slight modifications of the prior art device for manufacturing the prior art cotton pads, as it will be explained in the following, in connection with the different embodiments of the devices of the invention.

However, preferably, step c) of cutting the cotton webs B into the cotton pads and step d) of creating at least one precutting line throughout the cotton pad are carried out simultaneously.

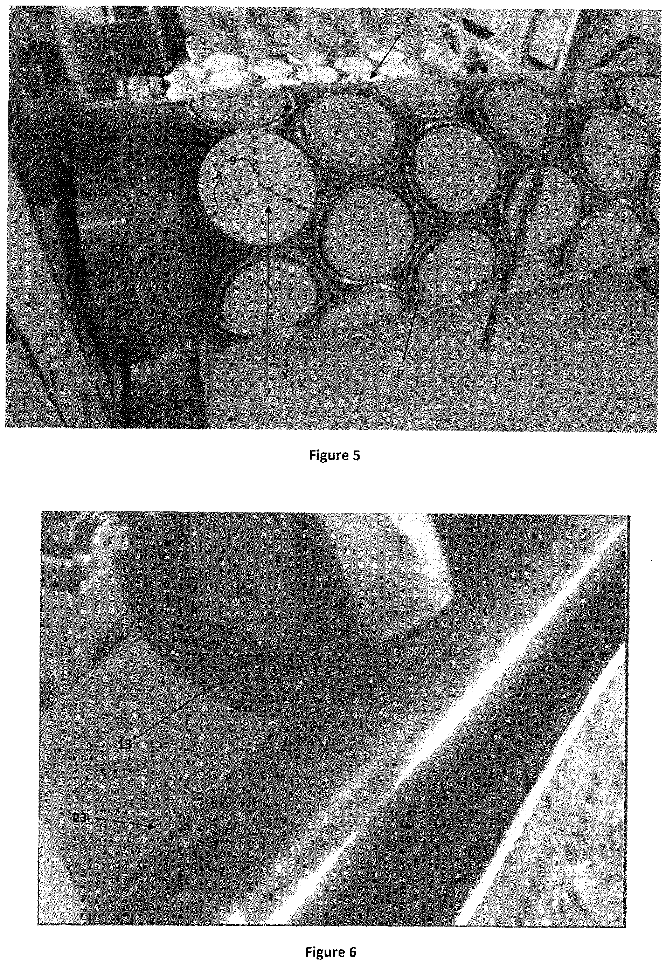

A device for implementing such a method in which the cotton pad is cut simultaneously with the creation of the precutting line is shown in figure 5.

As shown in figure 5, the device of the invention is a rotary cutting cylinder, noted 5 in figure 5, comprising knives, noted 6 in figure 5. In figure 5, the knives 6 are circular because the cotton pads to be produced are circular cotton pads.

It will clearly appear to the man skilled in the art that any other shapes or dimensions of the cotton pad may be obtained by varying the shapes and dimensions of the knives 6.

Into each knife 6, there is a second knife, noted 7 in figure 5, having the shape of the precutting line(s) to be obtained in the cotton pad. This knife 7 is a serrated knife. Otherwise stated, the surface of the knife 7 which is in contact with the cotton pad presents hollows, noted 8 in figure 5 and reliefs, noted 9 in figure 5. The distance between the end of each hollow 8 and the beginning of the following hollow 8 is the width w₂ of the cut part 4 of the precutting line to be obtained the cotton pad, and the width of the hollow 8 corresponds to the width w₁ of the link 3 to be obtained in the cotton pad.

However, as already stated, the at least one precutting line may be created in the cotton web before step c) of cutting the cotton pad and after step b) of unwinding the cotton web roll.

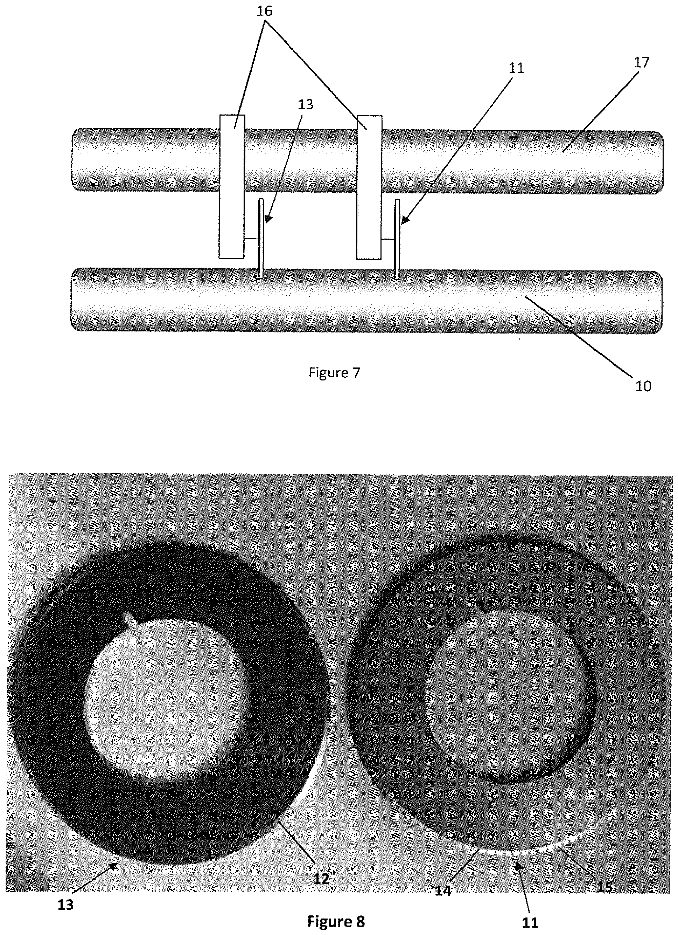

In this latter case, a first device for obtaining the cotton pad of the invention comprises:
- a rotary cylinder as shown in figure 5 comprising only knives 6, *i.e.* for cutting the cotton pads to the desired shape and dimensions but without knives 7, and
- upstream to this rotary cutting cylinder, a knife holder having only serrated knives, noted 11 in figure 8.

The serrated knives 11 have a cutting edge (the edge in contact with the webs B with hollows, noted 14 in figure 8), having width corresponding to the width w₁ of the links 3 to be obtained in the precutting line and reliefs, noted 15 in figure 8, having a width corresponding to the width w₂ of the cut parts 4 of the precutting line.

Thus, with this device (step b)), once the web B is unwounded, it is transported to the knife holder with serrated knife 11 where the precutting line(s) are formed (step d)) and then it is transported to the rotary cylinder for cutting the cotton pads (step c)).

As it will be apparent to the man skilled in the art, with this second device, the obtained cotton pads are pads having at least one precutting line.

Consequently, this precutting line is in the machine direction.

A third device of the invention is a device for forming at least one precutting line in the cotton web B after step b) of unwinding the cotton web rolls and before step c). With this device, the precutting line is formed in the cross direction machine.

The device is schematically represented in figure 9.

As it can be seen in figure 9, this device comprises a lower rotary cylinder, noted 18 in figure 9, for providing a counter pressure to the knives of the upper cylinder. The rotary cylinder 18 is situated face to an upper cylinder, noted 19 in figure 9, this rotary cylinder 19 having knives, noted 20 in figure 9, which are serrated knives, *i.e.* which comprises hollows, noted 22 in figure 9 and reliefs, noted 21 in figure 9.

The cutting knives 20 are perpendicular to the machine direction. More preferably, they form a slight angle of plus or minus 1° with the cross machine direction, *i.e.* of the axis noted X - X' of the rotary cylinder 19. In figure 9, this angle is widened for a better visualization.

This angle is for limiting the wear of the knives 20.

After webs B have been transported through the third device of the invention, they are transported to the cutting rotary cylinder where the cotton pads are cut to the desired shape and dimensions.

A fourth device of the invention is a device for carrying out the third embodiment of the process of the invention in which step d) of forming the at least one precutting line is carried out simultaneously with step a2) of slitting a cotton web A having a width L1 into webs B having a width L2 < L1 and winding the webs B into rolls.

This device is only a slight modification when compared to the device for manufacturing the cotton pads of the prior art for carrying out step a2).

The device of the prior art is shown in figure 6.

This device comprises a rotary cylinder, noted 23 in figure 6, and a cutting knife noted 13 in figure 6.

The cutting knife 13 is not serrated. It has, as shown on the left hand of figure 8, a cutting edge, noted 12 in figure 7, which is smooth.

Knife 13 is used for slitting the web A into the webs B. This slitting operation is carried out simultaneously with the operation of winding the webs B. Then, the webs B are transported to the cutting rotary cylinder where the cotton pads are cut to the desired shape and dimensions.

The fourth device of the invention itself is shown in figure 7.

The knife (knives) 11 and the knives 13 are attached to the cylinder 17 by knife holders noted 16 in figure 7.

This device also comprises a lower rotary cylinder noted 23 in figure 6 and 10 in figure 7 for providing a counter pressure to the knives 11 and 13. Once webs B have been cut, and, that the same time, the precutting line(s) have been formed, webs B are transported to another rotary cylinder comprising knives 6 for cutting the cotton pads to the desired shape and dimensions.

It comprises an upper cylinder, noted 17 in figure 7, comprising between two knives 13, at least one supplementary knife, noted 11 in figures 7 and 8. This supplementary knife 11 is a serrated knife, *i.e.* its cutting edge (the edge in contact with the web A and consequently the webs B) comprises hollows, noted 14 in figure 8, and reliefs, noted 15 in figure 8. The number of supplementary knives 11 depends on the number of pads to be cut in the widths L1 and L2 of the webs A and B.

The hollows 14 have the width w₁ of the links 3 to be obtained in the cotton pad and the reliefs 15 have the width w₂ of the cut parts 4 to be obtained in the cotton pad.

## Claims

1. A cotton pad (1) for skin care having at least one precutting line (2, 2', 2") consisting of links (3) and cut parts (4) delimiting at least two detachable parts of the pad, the two parts being maintained attached together by the links (3), wherein:
- the links (3) have a width w₁ of 0.8 mm, and
- the cut parts (4) have a width w₂ of 10mm.

2. The cotton pad of claim 1, wherein at least one of the precutting lines (2, 2") is formed in the cross machine direction.

3. The cotton pad of claim 1 or 2, having one precutting line (2) delimiting two detachable parts of the pad having the same dimensions.

4. The cotton pad of claim 1 or 2, having three cutting lines (2') delimiting three detachable parts of the pad having the same dimensions.

5. The cotton pad of claim 1 or 2, having two cutting lines (2") delimiting four detachable parts of the pad having the same dimensions.

6. The cotton pad of anyone of claims 1 to 5 which is impregnated with a skin care composition.

7. A method for producing a cotton pad according to anyone of claims 1-6, comprising the following steps:
a) providing a cotton web roll B,
b) unwinding the cotton web roll B of step a),
c) cutting the cotton web B obtained in step b) into cotton pads having the desired shape and dimensions,
and furthermore comprising a step d) of creating at least one precutting line throughout the cotton pad, preferably in the cross machine direction, wherein the at least one precutting line (2, 2', 2") consists of links (3) and cut parts (4) delimiting at least two detachable parts of the pad, the two parts being maintained attached together by the links (3),
- the links (3) have a width w₁ of 0.8 mm, and
- the cut parts (4) have a width w₂ of 10 mm.

8. The method of claim 7 further comprising before step a),
a step a1) of forming a cotton web A having a width L1 from cotton fibers or a mixture of cotton fibers and synthetic and/or natural fibers, and
a step a2) of slitting the cotton web A into cotton webs B having a width L2 < L1 and simultaneously winding the cotton webs B into rolls.

9. The method of claim 7 or 8, wherein steps c) and d) are carried out simultaneously.

10. The method of claim 7 or 8, wherein step d) is carried out before step c) and after step b).

11. The method of claim 8, wherein step a2) and step d) are carried out simultaneously.

12. A device configured for implementing the method of claim 9 for producing a cotton pad, comprising a rotary cutting cylinder (5) comprising knives (6) having the dimensions and shapes of the desired cotton pad and at least one serrated knife (7) inside each knife (6), the serrated knife (7) having hollows and cutting reliefs (9), the hollows (8) and cutting reliefs (9) having the dimensions of respectively the links (3) and cut parts (4) of the at least one precutting line (2, 2', 2") to be obtained in each individual cotton pad,
- the links (3) have a width w₁ of 0.8 mm, and
- the cut parts (4) have a width w₂ of 10 mm.

13. A device configured for implementing the method of claim 10 for producing a cotton pad with a precutting line in the machine direction, comprising:
- a rotary cutting cylinder comprising knives having the dimensions and shapes of the desired cotton pad, and
- a knife holder (16) placed upstream said rotary cutting cylinder,
said knife holder (16) holding at least one serrated knife (11) having hollows (14) and cutting reliefs (15), the hollows (14) and cutting reliefs (15) having the dimensions of respectively the links 3 and cut parts (4) of the at least one precutting line (2, 2', 2") to be obtained in each individual cotton pad,
- the links (3) have a width w₁ of 0.8 mm, and
- the cut parts (4) have a width w₂ of 10 mm.

14. A device configured for implementing the method of claim 11 for producing a cotton pad, comprising:
- an upper cylinder (17) holding:
- at least one knife (13) having a cutting edge (12) which is not serrated for slitting a web A having a width L1 into webs B having a width L2 < L1, and
- at least one serrated knife (11) having hollows (14) and cutting reliefs (15), the hollows (14) and cutting reliefs (15) having respectively the dimensions of the links (3) and cut parts (4) to be obtained in each individual cotton pad, and
- a rotary lower cylinder (10) for providing a counter pressure to the knives (11,13),
- the links (3) have a width w₁ of 0.8 mm, and
- the cut parts (4) have a width w₂ of 10 mm.

15. A device configured for implementing the method of claim 10 for producing a cotton pad with the precutting line in the cross machine direction, comprising:
- a rotary cylinder (19) comprising serrated knives (20) having hollows (22) and cutting reliefs (21) having respectively the dimensions of the links (3) and cut parts (4) of the at least one precutting line(s) to be obtained in the cotton pad, and
- a cylinder (18) for providing a counter pressure to the knives (20),
- the links (3) have a width w₁ of 0.8 mm, and
- the cut parts (4) have a width w₂ of 10 mm.

## Patentansprüche

1. Wattepad (1) zur Hautpflege, das mindestens eine Vorschneidelinie (2, 2', 2") aufweist, die aus Verbindungen (3) und geschnittenen Teilen (4) besteht, die mindestens zwei lösbare Teile des Pads abgrenzen, wobei die zwei Teile durch die Verbindungen (3) aneinander befestigt bleiben, wobei:
- die Verbindungen (3) eine Breite w₁ von 0,8 mm aufweisen, und
- die geschnittenen Teile (4) eine Breite w₂ von 10 mm aufweisen.

2. Wattepad nach Anspruch 1, wobei mindestens eine der Vorschneidelinien (2, 2") in der Quermaschinenrichtung gebildet ist.

3. Wattepad nach Anspruch 1 oder 2, das eine Vorschneidelinie (2) aufweist, die zwei lösbare Teile des Pads abgrenzt, welche die gleichen Abmessungen aufweisen.

4. Wattepad nach Anspruch 1 oder 2, das drei Schneidelinien (2') aufweist, die drei lösbare Teile des Pads abgrenzen, welche die gleichen Abmessungen aufweisen.

5. Wattepad nach Anspruch 1 oder 2, das zwei Schneidelinien (2") aufweist, die vier lösbare Teile des Pads abgrenzen, welche die gleichen Abmessungen aufweisen.

6. Wattepad nach einem der Ansprüche 1 bis 5, das mit einer Hautpflegezusammensetzung imprägniert ist.

7. Verfahren zum Herstellen eines Wattepads nach einem der Ansprüche 1-6, umfassend die folgenden Schritte:
a) Bereitstellen einer Wattebahnrolle B,
b) Abwickeln der Wattebahnrolle B von Schritt a),
c) Schneiden der in Schritt b) erhaltenen Wattebahn B in Wattepads, welche die erwünschte(n) Form und Abmessungen aufweisen,
und weiterhin umfassend einen Schritt d) des Erstellens mindestens einer Vorschneidelinie durch das ganze Wattepad, vorzugsweise in der Quermaschinenrichtung, wobei die mindestens eine Vorschneidelinie (2, 2', 2") aus Verbindungen (3) und geschnittenen Teilen (4) besteht, die mindestens zwei lösbare Teile des Pads abgrenzen, wobei die zwei Teile durch die Verbindungen (3) aneinander befestigt bleiben,
- die Verbindungen (3) eine Breite w₁ von 0,8 mm aufweisen, und
- die geschnittenen Teile (4) eine Breite w₂ von 10 mm aufweisen.

8. Verfahren nach Anspruch 7, weiter umfassend vor Schritt a),
einen Schritt a1) des Bildens einer Wattebahn A, die eine Breite L1 aus Wattefasern oder einem Gemisch von Wattefasern und synthetischen und/oder natürlichen Fasern aufweist, und
einen Schritt a2) des Schlitzens der Wattebahn A in Wattebahnen B, die eine Breite L2 < L1 aufweisen, und gleichzeitig des Wickelns der Wattebahnen B in Rollen.

9. Verfahren nach Anspruch 7 oder 8, wobei Schritte c) und d) gleichzeitig ausgeführt werden.

10. Verfahren nach Anspruch 7 oder 8, wobei Schritt d) vor Schritt c) und nach Schritt b) ausgeführt wird.

11. Verfahren nach Anspruch 8, wobei Schritt a2) und Schritt d) gleichzeitig ausgeführt werden.

12. Vorrichtung, konfiguriert zum Implementieren des Verfahrens nach Anspruch 9 zum Herstellen eines Wattepads, umfassend einen drehenden Schneidezylinder (5), der Klingen (6), welche die Abmessungen und Formen des erwünschten Wattepads aufweisen, und mindestens eine gezahnte Klinge (7) innerhalb jeder Klinge (6) umfasst, wobei die gezahnte Klinge (7) Vertiefungen und Schneideerhöhungen (9) aufweist, wobei die Vertiefungen (8) und Schneideerhöhungen (9) die Abmessungen von jeweils den Verbindungen (3) und geschnittenen Teilen (4) von der mindestens einen Vorschneidelinie (2, 2', 2") aufweisen, die in jedem einzelnen Wattepad zu erhalten sind,
- die Verbindungen (3) eine Breite w₁ von 0,8 mm aufweisen, und
- die geschnittenen Teile (4) eine Breite w₂ von 10 mm aufweisen.

13. Vorrichtung, konfiguriert zum Implementieren des Verfahrens nach Anspruch 10 zum Herstellen eines Wattepads mit einer Vorschneidelinie in der Maschinenrichtung, umfassend:
- einen drehenden Schneidezylinder, umfassend Klingen, welche die Abmessungen und Formen des erwünschten Wattepads aufweisen, und
- einen Klingenhalter (16), der stromaufwärts des drehenden Schneidezylinders platziert ist,
wobei der Klingenhalter (16) mindestens eine gezahnte Klinge (11) hält, die Vertiefungen (14) und Schneideerhöhungen (15) aufweist, wobei die Vertiefungen (14) und Schneideerhöhungen (15) die Abmessungen von jeweils den Verbindungen 3 und geschnittenen Teilen (4) von der mindestens einen Vorschneidelinie (2, 2', 2") aufweisen, die in jedem einzelnen Wattepad zu erhalten sind,
- die Verbindungen (3) eine Breite w₁ von 0,8 mm aufweisen, und
- die geschnittenen Teile (4) eine Breite w₂ von 10 mm aufweisen.

14. Vorrichtung, konfiguriert zum Implementieren des Verfahrens nach Anspruch 11 zum Herstellen eines Wattepads, umfassend:
- einen oberen Zylinder (17), der hält:
- mindestens eine Klinge (13), die eine Schneidekante (12), die nicht gezahnt ist, zum Schlitzen einer Bahn A, die eine Breite L1 aufweist, in Bahnen B, die eine Breite L2 < L1 aufweisen, aufweist, und
- mindestens eine gezahnte Klinge (11), die Vertiefungen (14) und Schneideerhöhungen (15) aufweist, wobei die Vertiefungen (14) und Schneideerhöhungen (15) jeweils die Abmessungen der Verbindungen (3) und geschnittenen Teile (4) aufweisen, die in jedem einzelnen Wattepad zu erhalten sind, und
- einen drehenden unteren Zylinder (10) zum Bereitstellen eines Gegendrucks an die Klingen (11, 13),
- die Verbindungen (3) eine Breite w₁ von 0,8 mm aufweisen, und
- die geschnittenen Teile (4) eine Breite w₂ von 10 mm aufweisen.

15. Vorrichtung, konfiguriert zum Implementieren des Verfahrens nach Anspruch 10 zum Herstellen eines Wattepads mit der Vorschneidelinie in der Quermaschinenrichtung, umfassend:
- einen drehenden Zylinder (19), umfassend gezahnte Klingen (20), die Vertiefungen (22) und Schneideerhöhungen (21) aufweisen, die jeweils die Abmessungen der Verbindungen (3) und geschnittenen Teile (4) von der mindestens einen Vorschneidelinie(n) aufweisen, die in dem Wattepad zu erhalten sind, und
- einen Zylinder (18) zum Bereitstellen eines Gegendrucks an die Klingen (20),
- die Verbindungen (3) eine Breite w₁ von 0,8 mm aufweisen, und
- die geschnittenen Teile (4) eine Breite w₂ von 10 mm aufweisen.

## Revendications

1. Tampon de coton (1) pour le soin de la peau ayant au moins une ligne de prédécoupe (2, 2', 2") constituée de parties de liaison (3) et parties découpées (4) délimitant au moins deux parties détachables du tampon, les deux parties étant maintenues attachées ensemble par les parties de liaison (3), dans lequel :
- les parties de liaison (3) ont une largeur w₁ de 0,8 mm, et
- les parties découpées (4) ont une largeur w₂ de 10 mm.

2. Tampon de coton selon la revendication 1, dans lequel au moins une des lignes de prédécoupe (2, 2") est formée dans la direction transversale à la machine.

3. Tampon de coton selon la revendication 1 ou 2, ayant une ligne de prédécoupe (2) délimitant deux parties détachables du tampon ayant les mêmes dimensions.

4. Tampon de coton selon la revendication 1 ou 2, ayant trois lignes de prédécoupe (2') délimitant trois parties détachables du tampon ayant les mêmes dimensions.

5. Tampon de coton selon la revendication 1 ou 2, ayant deux lignes de prédécoupe (2") délimitant quatre parties détachables du tampon ayant les mêmes dimensions.

6. Tampon de coton selon l'une quelconque des revendications 1 à 5 qui est imprégné d'une composition de soins de la peau.

7. Procédé pour produire un tampon de coton selon l'une quelconque des revendications 1 à 6, comprenant les étapes suivantes :
a) la fourniture d'un rouleau de bande de coton B,
b) le déroulement du rouleau de bande de coton B de l'étape a),
c) la découpe de la bande de coton B obtenue à l'étape b) en tampons de coton ayant la forme et les dimensions souhaités,
et comprenant en outre une étape d) de création d'au moins une ligne de prédécoupe à travers le tampon de coton, de préférence dans la direction transversale à la machine, dans lequel l'au moins une ligne de prédécoupe (2, 2', 2") est constituée de parties de liaison (3) et de parties découpées (4) délimitant au moins deux parties détachables du tampon, les deux parties étant maintenues attachées ensemble par les parties de liaison (3),
- les parties de liaison (3) ont une largeur w₁ de 0,8 mm, et
- les parties découpées (4) ont une largeur w₂ de 10 mm.

8. Procédé selon la revendication 7 comprenant en outre avant l'étape a),
une étape a1) de formation d'une bande de coton A ayant une largeur L1 à partir de fibres de coton ou d'un mélange de fibres de coton et de fibres synthétiques et/ou naturelles, et
une étape a2) de fendage de la bande de coton A en tampons de coton B ayant une largeur L2 < L1 et d'enroulement simultané des bandes de coton B en rouleaux.

9. Procédé selon la revendication 7 ou 8, dans lequel les étapes c) et d) sont réalisées simultanément.

10. Procédé selon la revendication 7 ou 8, dans lequel l'étape d) est réalisée avant l'étape c) et après l'étape b).

11. Procédé selon la revendication 8, dans lequel l'étape a2) et l'étape d) sont réalisées simultanément.

12. Dispositif configuré pour mettre en oeuvre le procédé de la revendication 9 pour produire un tampon de coton, comprenant un cylindre de découpe rotatif (5) comprenant des couteaux (6) ayant les dimensions et formes du tampon de coton souhaité et au moins un couteau dentelé (7) à l'intérieur de chaque couteau (6), le couteau dentelé (7) ayant des creux et des reliefs de découpe (9), les creux (8) et reliefs de découpe (9) ayant les dimensions de respectivement les parties de liaison (3) et les parties découpées (4) de l'au moins une ligne de prédécoupe (2, 2', 2") à obtenir dans chaque tampon de coton individuel,
- les parties de liaison (3) ont une largeur w₁ de 0,8 mm, et
- les parties découpées (4) ont une largeur w₂ de 10 mm.

13. Dispositif configuré pour mettre en oeuvre le procédé de la revendication 10 pour produire un tampon de coton avec une ligne de prédécoupe dans la direction de machine, comprenant :
- un cylindre de découpe rotatif comprenant des couteaux ayant les dimensions et formes du tampon de coton souhaité, et
- un support de couteau (16) placé en amont dudit cylindre de découpe rotatif,
ledit support de couteau (16) supportant au moins un couteau dentelé (11) ayant des creux (14) et reliefs de découpe (15), les creux (14) et reliefs de découpe (15) ayant les dimensions de respectivement les parties de liaison (3) et les parties découpées (4) de l'au moins une ligne de prédécoupe (2, 2', 2") à obtenir dans chaque tampon de coton individuel,
- les parties de liaison (3) ont une largeur w₁ de 0,8 mm, et
- les parties découpées (4) ont une largeur w₂ de 10 mm.

14. Dispositif configuré pour mettre en oeuvre le procédé de la revendication 11 pour produire un tampon de coton, comprenant :
- un cylindre supérieur (17) supportant :
- au moins un couteau (13) ayant un bord de découpe (12) qui n'est pas dentelé pour fendre une bande A ayant une largeur L1 en bandes B ayant une largeur L2 < L1, et
- au moins un couteau dentelé (11) ayant des creux (14) et reliefs de découpe (15), les creux (14) et reliefs de découpe (15) ayant respectivement les dimensions des parties de liaison (3) et des parties de découpe (4) à obtenir dans chaque tampon de coton individuel, et
- un cylindre inférieur rotatif (10) pour fournir une contre-pression aux couteaux (11, 13),
- les parties de liaison (3) ont une largeur w₁ de 0,8 mm, et
- les parties découpées (4) ont une largeur w₂ de 10 mm.

15. Dispositif configuré pour mettre en oeuvre le procédé de la revendication 10 pour produire un tampon de coton avec la ligne de prédécoupe dans la direction transversale à la machine, comprenant :
- un cylindre rotatif (19) comprenant des couteaux dentelés (20) ayant des creux (22) et reliefs de découpe (21) ayant respectivement les dimensions des parties de liaison (3) et des parties découpées (4) de l'au moins une ligne de prédécoupe à obtenir dans le tampon de coton, et
- un cylindre (18) pour fournir une contre-pression aux couteaux (20),
- les parties de liaison (3) ont une largeur w₁ de 0,8 mm, et
- les parties découpées (4) ont une largeur w₂ de 10 mm.
